(19) Europäisches Patentamt
European Patent Office
Office européen des brevets

(11) **EP 2 866 606 B1**

(12) **FASCICULE DE BREVET EUROPEEN**

(45) Date de publication et mention
de la délivrance du brevet:
**10.04.2019 Bulletin 2019/15**

(51) Int Cl.:
***A45D 34/04*** *(2006.01)*     ***A61M 35/00*** *(2006.01)*

(21) Numéro de dépôt: **13747359.1**

(22) Date de dépôt: **01.07.2013**

(86) Numéro de dépôt international:
**PCT/EP2013/063817**

(87) Numéro de publication internationale:
**WO 2014/005988 (09.01.2014 Gazette 2014/02)**

(54) **APPLICATEUR DE PRODUIT FLUIDE TEL QU'UN PRODUIT COSMÉTIQUE**

APPLIKATOR FÜR FLÜSSIGE SUBSTANZEN, INSBESONDERE FÜR KOSMETIKA

APPLICATOR FOR FLUID PRODUCTS SUCH AS COSMETICS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.07.2012 FR 1256307**

(43) Date de publication de la demande:
**06.05.2015 Bulletin 2015/19**

(73) Titulaire: **COSMOGEN**
**75008 Paris (FR)**

(72) Inventeurs:
• **GIEUX, Gérard**
**B-1180 Bruxelles (BE)**
• **BONNEYRAT, Philippe**
**F-95220 Herblay (FR)**

(74) Mandataire: **Gaillarde, Frédéric F. Ch. et al**
**Cabinet Germain & Maureau**
**31-33, rue de la Baume**
**75008 Paris (FR)**

(56) Documents cités:
**WO-A2-2011/083427     FR-A1- 2 915 972**

**Description**

La présente invention se rapporte à un applicateur de produit fluide, tel qu'un produit cosmétique, par exemple pour l'application sur la peau, dont la tête d'application et de distribution de produit propose un effet thermique, afin de créer une sensation de froid à l'application.

Par produit cosmétique, on entend un produit tel que défini dans la Directive 93/35/CEE du Conseil du 14 juin 1993.

Il est en effet souhaitable, dans certains cas, d'associer une sensation thermique à l'application d'un produit cosmétique afin, notamment, d'en accroître l'effet.

Tel est le cas, notamment, des produits pour le contour des yeux pour lesquels les fabricants de produits cosmétiques considèrent que le fait de procurer un effet froid sur la peau, au cours de l'application du produit, permet de diminuer les rides, d'effacer les poches, d'atténuer les traces de fatigue, de raffermir ou retendre la peau et /ou de paraître momentanément plus jeune.

De tels avantages sont liés à l'effet de constriction momentanée des vaisseaux sanguins, entraînant une baisse du débit sanguin lorsque la peau entre en contact d'une matière procurant une sensation de froid lors de l'application.

Plusieurs dispositifs permettant de distribuer un produit cosmétique et/ou de masser une surface corporelle sont connus pour offrir de telles propriétés.

Le document FR 2 915 972 enseigne d'utiliser l'effusivité thermique des matériaux constituant l'applicateur pour créer une sensation de froid à l'application.

Plus particulièrement, il divulgue un dispositif de distribution de produit cosmétique comprenant notamment :

- un boîtier ayant un réservoir pour contenir le produit ;
- un embout de stockage thermique couplé au boîtier, l'embout de stockage thermique ayant une face d'application pour appliquer le produit sur une surface et,
- un insert disposé dans l'embout de stockage thermique, l'insert définissant une voie de passage de distribution de produit en communication avec le réservoir, la voie de passage de distribution de produit s'étendant à travers l'embout de stockage thermique et se terminant par une ouverture dans la face d'application.

Par embout de stockage thermique, on entend un embout fabriqué dans un matériau dont l'effusivité thermique est très supérieure à celle de la zone traitée par l'applicateur correspondant.

L'Effusivité Thermique est la capacité d'un matériau à absorber les calories.

Plus sa valeur est élevée, plus le matériau est apte à absorber les calories sans s'échauffer, et inversement.

La valeur intrinsèque d'Effusivité Thermique d'un matériau se calcule de la manière suivante :

$$Ef = \sqrt{k.\rho.c}$$

où

k est la capacité thermique en $W.m^{-1}.°K^{-1}$ ; $\rho$ est la masse volumique en $kg/m^3$ ; c est la conductivité thermique en $J.kg^{-1}.°K^{-1}$.

Ainsi, au contact de la peau, alors que les pièces sont toutes à la même température, une pièce en acier (dont l'Effusivité est de l'ordre de 14 000 $J.K^{-1}.m^{-2}.s^{-1/2}$) donnera une sensation de froid sur la peau (Effusivité de 400 $J.K^{-1}.m^{-2}.s^{-1/2}$), alors qu'une pièce en bois (Effusivité également de l'ordre de 400 $J.K^{-1}.m^{-2}.s^{-1/2}$) donnera une sensation neutre.

Ce transfert de chaleur donne sur la peau une sensation de froid d'autant plus intense que l'Effusivité Thermique est élevée.

Or, de tels dispositifs présentent de nombreux inconvénients.

En premier lieu, l'utilisateur n'a pas la possibilité de s'affranchir de la sensation de froid procurée par le contact de l'applicateur sur la peau d'un utilisateur lors d'une utilisation du dispositif de distribution de produit correspondant.

Or, cette sensation de froid n'est, souvent, pas nécessaire à chaque utilisation du dispositif de distribution de produit ou non désirée. L'utilisateur peut vouloir ne profiter que du produit cosmétique, sans nécessairement subir un contact glacé qui peut être désagréable dans certaines circonstances.

Par ailleurs, pour procurer une sensation de froid qui ne soit pas trop éphémère, il convient d'avoir une surface à forte effusivité thermique suffisamment importante. On a tendance à augmenter, dès lors, la surface enveloppe de la face d'application de l'embout de stockage thermique.

Or, la distribution du produit étant réalisée par une ouverture débouchant dans cette face d'application, une surface trop importante nuit à la précision de la distribution du produit, notamment sur les parties sensibles de type contour des yeux.

De tels dispositifs sont, souvent, inadaptés pour distribuer un produit cosmétique sur des surfaces non planes, complexes, présentant à la fois des concavités et des convexités tels que le contour des yeux, cette application devant se faire de manière très précise pour éviter tout risque d'irritation des yeux par le produit cosmétique.

Par ailleurs, avec des imprécisions dans la distribution du produit, l'utilisateur tend à distribuer et consommer une quantité plus importante de produit contenu dans le réservoir.

Il existe un besoin pour gagner en précision dans le choix de la sensation procurée par l'application et/ou dans la distribution et l'application d'un produit cosmétique sur une zone à traiter.

Ainsi, un but de la présente invention est de proposer un applicateur dans lequel le choix de bénéficier ou non des propriétés réfrigérantes de l'applicateur est laissé à l'utilisateur.

Il est également désirable de proposer un applicateur dans lequel on contrôle la distribution du produit sur la zone à traiter, ceci afin d'améliorer la précision du lieu de distribution et la quantité de produit distribué.

A cet effet, l'invention propose un applicateur de produit fluide selon la revendication 1 tel qu'un produit liquide, semi-liquide ou visqueux comprenant un réservoir contenant le produit et une tête d'application, couplée au réservoir, comprenant :

- un embout de distribution du produit en communication avec le réservoir comportant une face de distribution du produit sur une surface à traiter telle que la peau d'un utilisateur, cet embout définissant une voie de distribution dudit produit en communication avec le réservoir, la voie de distribution du produit s'étendant à travers l'embout de distribution et se terminant par un orifice sur la face de distribution, adapté pour distribuer le produit sur la surface à traiter,
- un embout de stockage thermique comportant une face d'application sur la surface à traiter.

L'applicateur est remarquable en ce que :

- l'embout (200) de stockage thermique est formé d'un matériau dont l'effusivité thermique est supérieure à celle de l'embout (100) de distribution de manière à procurer deux sensations thermiques différentes en contact avec la surface à traiter et,
- la tête d'application est conformée de sorte que, en orientant l'applicateur de façon sélective relativement à la surface à traiter, un utilisateur peut placer cette dernière en contact avec la face de distribution de l'embout de distribution tout en excluant un contact avec la face d'application de l'embout de stockage thermique.
- les faces de distribution et d'application respectives de l'embout de distribution et l'embout de stockage thermique sont définies par des surfaces enveloppes respectives non incluses l'une dans l'autre et disjointes ;

Selon des modes particuliers de réalisation de l'invention, un applicateur de produit fluide selon l'invention peut comprendre une ou plusieurs des caractéristiques suivantes, prises isolément ou en combinaison techniquement possibles :

- la face d'application est décalée relativement à la face de distribution le long d'un axe longitudinal de l'applicateur et/ou angulairement autour de la périphérie dudit applicateur ;
- une rupture de pente et/ou un décrochement sont ménagés entre les faces de distribution et d'application;
- l'embout de stockage thermique et l'embout de distribution forment deux quartiers complémentaires du contour externe de l'applicateur sur au moins une partie de la longueur de ce dernier ;
- l'embout de stockage thermique s'étend sur une première partie du contour périphérique externe de l'applicateur et l'embout de distribution s'étend sur une deuxième partie du contour périphérique de l'applicateur, de façon complémentaire à l'embout de stockage thermique;
- l'embout de distribution présente une forme de canule longiligne terminée par l'orifice de distribution;
- l'orifice de distribution est déporté à la périphérie externe de la face d'application de l'embout de stockage thermique et/ou en saillie de la tête d'application ;
- l'embout de stockage thermique est formé par une bague munie d'une face d'application bombée, adaptée pour être montée, de façon amovible, sur l'embout de distribution.

D'autres caractéristiques, buts et avantages de la présente invention, apparaîtront à la lecture de la description détaillée qui va suivre, selon les modes de réalisation donnés à titre d'exemples non limitatifs, et en référence aux dessins annexés sur lesquels :

- les figures 1a, 1b, 1c, 1d sont des vues, respectivement, en perspective éclatée, en coupe longitudinale, en perspective et latérale d'un premier mode de réalisation d'un applicateur selon la présente invention ;
- les figures 2a, 2b, 2c sont des vues, respectivement, en perspective éclatée, latérale et en perspective d'un second mode de réalisation d'un applicateur selon la présente invention ;
- les figures 3a, 3b, 3c, 3d sont des vues partielles, respectivement, en perspective éclatée, en coupe longitudinale, en perspective et latérale d'un troisième mode de réalisation d'un applicateur selon la présente invention ;
- les figures 4a, 4b, 4c sont des vues, respectivement, en perspective éclatée, latérale et en perspective d'un quatrième mode de réalisation d'un applicateur selon la présente invention ;
- les figures 5a et 5b sont des vues, respectivement latérale et en perspective d'un cinquième mode de réalisation d'un applicateur selon la présente invention ;
- les figures 6a, 6b, 6c sont des vues, respectivement, en en coupe longitudinale et deux vues latérales d'un sixième mode de réalisation d'un applicateur selon la présente invention ;
- les figures 7a, 7b, 7c sont des vues, respectivement, en perspective éclatée, en coupe longitudinale, en perspective d'un septième mode de réalisation d'un applicateur selon la présente invention ;

- les figures 8a, 8b, 8c sont des vues partielles, respectivement, en perspective éclatée, en perspective et latérale d'un huitième mode de réalisation d'un applicateur selon la présente invention.

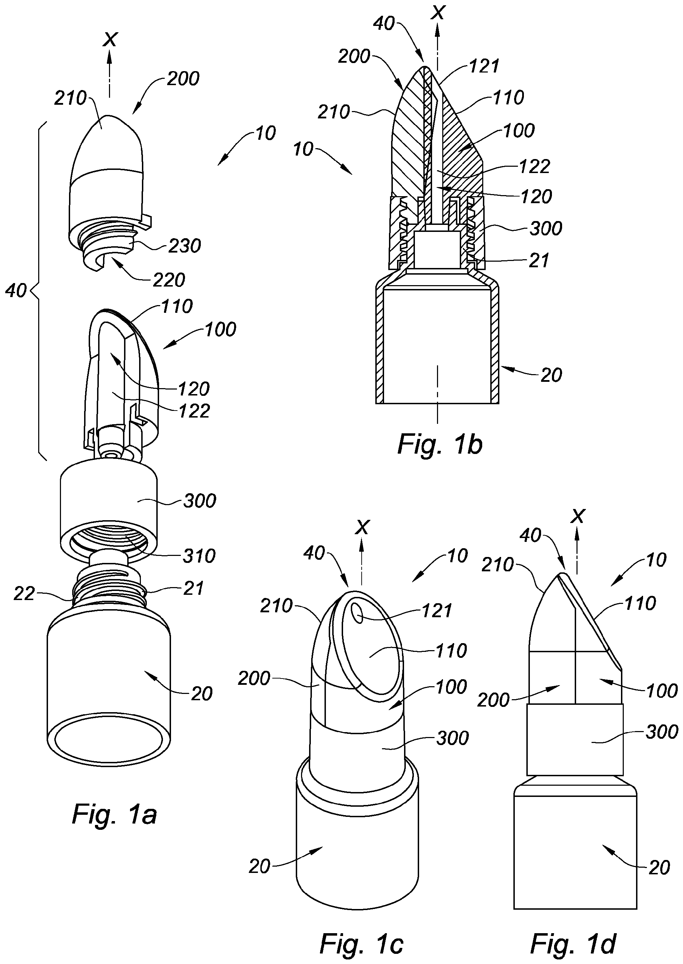

On se reporte à présent aux figures 1a à 1d, sur lesquelles on oberve un applicateur 10 de produit fluide tel qu'un produit liquide, semi-liquide ou visqueux contenu dans un récipient 20 ou réservoir et destiné à être appliqué au moyen d'une tête d'application 40 couplée à une extrémité du réservoir 20. Cet applicateur 10 s'étend longitudinalement selon un axe longitudinal X.

Le cas échéant, la tête d'application 40 coopère avec un capuchon de fermeture 30 de l'applicateur 10, qui n'est pas visible sur ces figures. Il sera décrit ci-après plus en détail.

Plus particulièrement, cet applicateur 10 comprend une tête d'application 40 formée par :

- un embout 100 dit de distribution du produit en communication avec le réservoir 20, comprenant une face 110 de distribution active du produit sur une surface à traiter d'un utilisateur telle que la peau.

Cet embout 100 définit une voie 120 de passage de distribution dudit produit en communication avec le réservoir 20, la voie 120 de passage de distribution du produit s'étendant à travers l'embout 100 de distribution et se terminant par une ouverture 121 sur la face 110 de distribution, adaptée pour distribuer le produit sur la surface à traiter ;

- un embout 200 dit de stockage thermique comprenant une face 210 d'application active sur la surface à traiter.

Par " face active" 110,210, on entend la surface de chaque embout 100,200 qui est destinée à être appliquée sur la surface à traiter de l'utilisateur, dans le cadre d'une utilisation de l'applicateur 10 conforme à sa destination.

L'embout de distribution 100 et, plus particulièrement, la face de distribution 110 est réalisé dans une matière dont l'effusivité thermique est proche de celle de la surface à traiter de l'utilisateur, procurant dès lors une sensation neutre à l'utilisateur.

L'embout 200 de stockage thermique et, plus particulièrement, la face d'application 210, est réalisé dans une matière à forte effusivité thermique au regard de celle de la surface à traiter de l'utilisateur.

L'effusivité thermique de la face d'application 210 active de l'embout de stockage thermique 200 est donc plus élevée que celle de la face de distribution 110 active de l'embout de disitrbution du produit 100.

De plus, la face d'application 210 est adaptée pour exercer, le cas échéant, un massage de la zone traitée.

Avantageusement, la tête d'application 40 est conformée de sorte que, en orientant l'applicateur 10 de façon sélective relativement à la surface à traiter, un utilisateur peut placer cette dernière en contact avec la face 110 de distribution de l'embout 100 de distribution tout en excluant un contact avec la face d'application 210 de l'embout 200 de stockage thermique.

Ainsi, les deux embouts de distribution 100 et de stockage thermique 200 sont conformés de sorte qu'en appliquant l'applicateur 20 sur une surface plane de la peau d'un utilisateur, la face 110 de distribution active peut distribuer et appliquer le produit sur la surface traitée, sans que la surface traitée ait un contact simultané avec la face 210 d'application de l'embout de stockage thermique 200 susceptible de procurer une sensation de froid à ladite surface.

Il faut, dès lors, effectuer une manoeuvre volontaire de la main pour tourner l'applicateur 10, afin de changer son orientation relativement à la zone de peau traitée et profiter volontairement de l'effet thermique sur cette zone.

Dans ce cadre, à la différence de l'art antérieur considéré, les faces 110 de distribution et d'application 210 actives respectives de l'embout 100 de distribution et l'embout 200 de stockage thermique sont définies par des surfaces enveloppes respectives non incluses l'une dans l'autre et disjointes.

Par surface enveloppe d'une face, on entend l'ensemble de la surface active de la face d'application considérée, destinée à être appliquée sur la surface à traiter de l'utilisateur dans le cadre d'une utilisation de l'applicateur 10 conforme à sa destination.

Chaque embout de distibution 100 et de stockage thermique 200 peut présenter toute conformation qui respecte les propriétés de l'applicateur 10 ci-dessus.

Les configurations relatives des deux faces 110 de distribution et d'application 210 des deux embouts 100, 200 peuvent être non incluses l'une dans l'autre et disjointes de différentes façons.

On peut prévoir, notamment, de ménager une rupture de pente, un décrochement, un décalage axial ou angulaire entre ou sur l'un ou l'autre des deux faces 110,210 actives considérées ou surface enveloppes considérées.

Plus particulièrement, la face 210 d'application de l'embout de stockage thermique 200 est décalée relativement à la face de distribution 110 de l'embout de distribution 100 le long de l'axe X de l'applicateur 10 et/ou angulairement autour de la périphérie dudit applicateur 10.

Ainsi, l'utilisateur place la surface à traiter en contact, de façon sélective :

- soit avec la face 110 de distribution de l'embout de distribution 100, ceci afin de distribuer et appliquer le produit sur la surface traitée, en excluant toute sensation de froid lors de la distribution et l'application initiale du produit ;

- soit avec la face 210 d'application de l'embout 200 de stockage thermique, le cas échéant, s'il souhaite bénéficier d'une sensation de froid, ceci après la distribution et l'application initiale du produit sur la surface traitée.

La forme de chaque embout 100,200 et, plus particulièrement, les caractéristiques de chacune des faces de distribution 110 et d'application 210 peut être différente en fonction du produit à appliquer ou du type d'application.

Chacune d'elle peut être, notamment mais non exclusivement, de type sphérique, curviligne, parabolique, plane, convexe, en ogive ou constituer une combinaison de formes connues.

Différentes variantes de réalisation des embouts de distribution 100 et de stockage thermique 200 sont illustrées sur les figures 1 à 8.

En référénce aux figures 1 à 7, on observe des applicateurs 20 dans lesquels, en section transversale de l'applicateur 10, l'embout 200 de stockage thermique et l'embout 100 de distribution forme deux quartiers complémentaires du contour externe de l'applicateur 10 sur au moins une partie de la longueur de ce dernier.

Sur ces figures, l'embout 200 de stockage thermique s'étend sur une première partie du contour périphérique de l'applicateur 20 et l'embout de distribution 100 s'étend sur une deuxième partie du contour périphérique de l'applicateur 10, de façon complémentaire à l'embout 200 de stockage thermique.

En référence plus particulièrement aux figures 1a à 1d, dans une première variante de réalisation, la tête d'application 40 présente une surface d'enveloppe de forme assimilable à un paraboloïde biseauté à son extrémité supérieure.

Le biseau se confond avec la face de distribution 110 du produit et forme un plan incliné par rapport à l'axe X de l'applicateur 10.

La voie 120 de passage du produit du réservoir 20 vers la face de distribution 110 est ménagée grâce à un canal 122 de distribution de l'embout 100 s'étendant le long de l'axe X et débouchant sur l'orifice 121 ménagé sur la partie supérieure de la face 110 biseautée.

La face de distribution 110 biseautée définit une surface d'application sensiblement plane qui permet d'appliquer le produit sur la surface à traiter.

Par ailleurs, le canal 122 de distribution peut se présenter sous la forme d'un ou plusieurs éléments tubulaires en communication avec un ou plusieurs réservoirs 20.

Il peut, également, présenter une section constante ou non, voire être adapté pour déboucher sur plusieurs orifices 121 sur la face 110 biseautée de l'embout 100.

Concernant l'embout 200 de stockage thermique, il est conformé comme la fraction complémentaire de celle de l'embout 100 de distribution.

En section transversale de l'applicateur 10, sur une partie de la longueur de la tête d'application 40, l'embout 200 de stockage thermique présente une section en forme de C coopérant avec une section de forme complémentaire de l'embout de distribution 100.

Cet embout de stockage thermique 200 est muni d'une gorge 220 longitudinale ouverte dont la concavité est orientée en direction du canal 122 de l'embout 200 de distribution et dont la forme et les dimensions sont adaptées pour recevoir ce canal 122.

L'extrémité supérieure 201 de l'embout 200 de stockage thermique, opposée au réservoir 20 est, quant à elle, conformée pour épouser une face interne de la face de distribution 110 biseautée sur une partie de sa périphérie externe.

Ainsi, la face d'application 210 active de l'embout 200 de stockage thermique est formée par la surface externe incurvée de l'embout 200.

Dès lors, la face active de distribution 110 est séparée de la face active d'application 210 par une arête courbe.

Il est, par conséquent, nécessaire d'effectuer une rotation autour d'un axe incliné par rapport à l'axe X pour passer de la distribution et l'application initiale du produit sur la peau de l'utilisateur à une application /massage du produit procurant une sensation de froid sur la peau de l'utilisateur, lors d'une utilisation de l'applicateur 20.

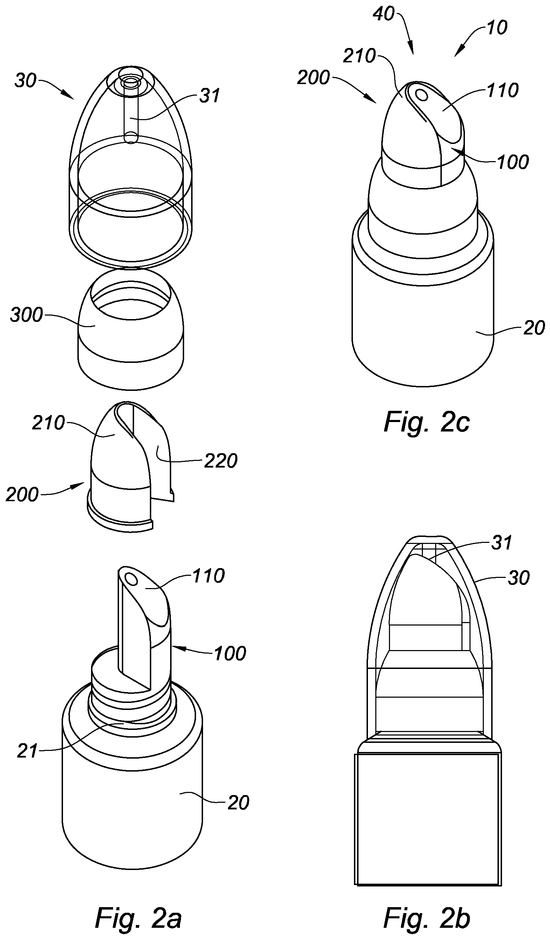

Dans la variante de réalisation illustrée sur les figures 2a à 2c, les embouts de distribution 100 et de stockage thermique 200 sont identiques à ceux décrits aux figures 1a à 1d aux différences suivantes.

L'embout de distribution 100 présente une section transversale triangulaire arrondie aux sommets.

L'embout 200 de stockage thermique, quant à lui, présente une section transversale complémentaire à celle de l'embout de distribution 100. La forme et les dimensions de la la gorge 220 longitudinale sont, ainsi, adaptées pour recevoir l'embout de distribution 100.

Le biseau de la tête d'application 40 est de section triangulaire, formé uniquement par la face de distribution active 110 de l'embout de distribution 100.

De façon similaire aux figures 1, l'orifice de distribution 121 est ménagé en partie supérieure de la face de distribution 110.

Afin d'isoler la face de distribution active 110 de la surface externe de l'embout, cette face de distribution 110 est conformée de manière à surmonter la surface externe de l'embout de stockage thermique 200.

Il y a, par conséquent, une rupture de pente entre la face de distribution 110 et la périphérie externe correspondante de l'embout de stockage thermique 200 qui forme avec le reste de la surface externe de l'embout, la face d'application active 210.

On s'assure, dès lors, l'absence de contact avec la matière au contact froid, au moment de l'application initiale du produit sur la zone traitée.

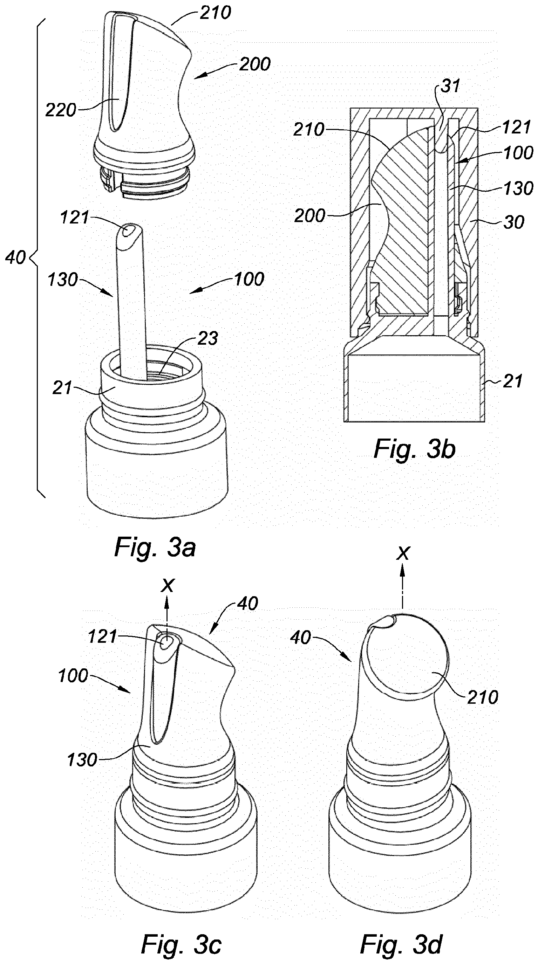

En référence aux figures 3a à 3d, la tête d'application 40 présente une forme générale de cylindre dont l'extrémité supérieure, destinée à être orientée à l'opposé du réservoir 2 (non visible sur ces figures) prend la forme d'un biseau.

L'embout de distribution 100 présente une forme de canule 130 longiligne terminée, à son extrémité opposée au réservoir 20, par l'orifice de distribution 121.

Dès lors, la face de distribution 110 est réduite à l'orifice de distribution 121.

Une telle face de distribution réduite tout comme le fait de déporter l'orifice de distribution 121 à la périphérie externe de la face d'application 210 et/ou en saillie de la tête d'application 40 permet, avantageusement, d'améliorer la précision de la distribution du produit sur la surface à traiter, notamment dans des zones du visage complexes tel que le contour des yeux.

On réduit, dès lors, les risques d'infection de parties sensibles à traiter par le produit.

De plus, une précision dans la distribution de produit entraîne une économie sur la quantité de produit distribué. Les excès de distribution de produit sont maîtrisés.

L'embout de stockage thermique 200 entoure, partiellement, cette canule 130 et la loge dans la gorge 220 longitidinale ouverte de forme et de dimensions adaptées.

Par ailleurs, cet embout 200 présente une face d'application 210 biseautée, de section circulaire, formant un plan incliné relativement à l'axe X.

L'orifice de distribution 121 est ménagé à la périphérie externe de cette face d'application 210, décalé axialement selon l'axe X à une altitude légèrement supérieure pour former une pointe d'extrémité de l'applicateur 20.

L'orifice de distribution 121 est, de plus, incliné dans une direction symétrique relativement à un plan P longitudinal de celle de la face d'application 210 biseautée.

Il est décalé angulairement autour de l'axe X de la face d'application 210.

Ainsi, la face de distribution 110 active est disjointe de la face d'application 210 de l'embout de stockage thermique 200.

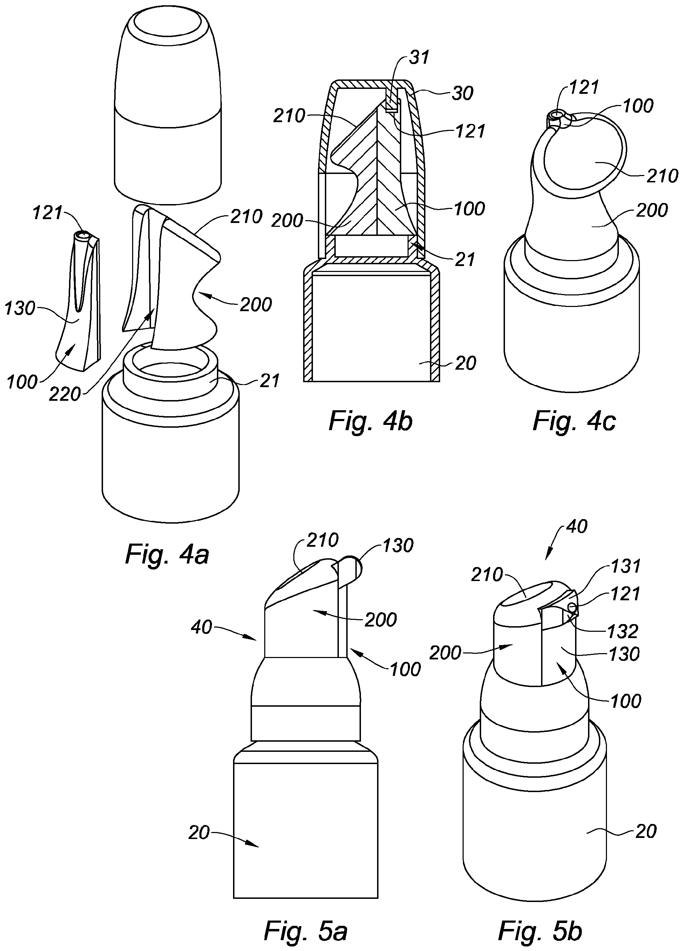

La tête d'application 40 de la variante de réalisation illustré sur les figures 4a à 4c diffère de celle illustrée sur les figures 3 en ce que l'orifice de distribution 121 n'est plus incliné mais conformé comme une surface plane perpendiculaire à l'axe X.

La propriété disjointe de la face de distribution 110 relativement à la face d'application 210 de l'embout de stockage thermique 200 est assurée par un décalage axial le long de l'axe X de l'orifice de distribution 121 relativement au contour externe de la face d'application 210.

L'orifice de distribution 121 fait saillie axialement à la périphérie externe de la face d'application 210 où il est déporté et forme une pointe d'extrémité latérale de l'applicateur 10.

De plus, la canule 130 est distincte du réservoir 20 alors que sur les figures 4, la canule 130 est une extension du réservoir 20.

Une telle variante de réalisation d'une tête d'application 40 permet, également, de favoriser une distribution précise du produit sur les zones traitées de l'utilisateur.

Tel est le cas, également, de la variante de réalisation illustrée sur les figures 5a et 5b qui diffère de la variante des figures 3 en ce que la canule 130 de l'embout de distribution 100 est coudée.

La branche supérieure 131 de la canule 130 est dirigée dans une direction opposée à celle de la face d'application 210 de l'embout de stockage thermique 200.

La canule 130 est, ainsi, conformée de sorte que l'orifice de distribution 121 fait saillie latéralement de la tête d'application 40, à la périphérie externe supérieure de la face d'application 210 de l'embout de stockage thermique 200.

Dans un exemple non limitatif illustré dans cette variante, l'extrémité libre de cette canule 130 prend la forme d'une pyramide 132 dans laquelle est ménagé l'orifice de distribution 121.

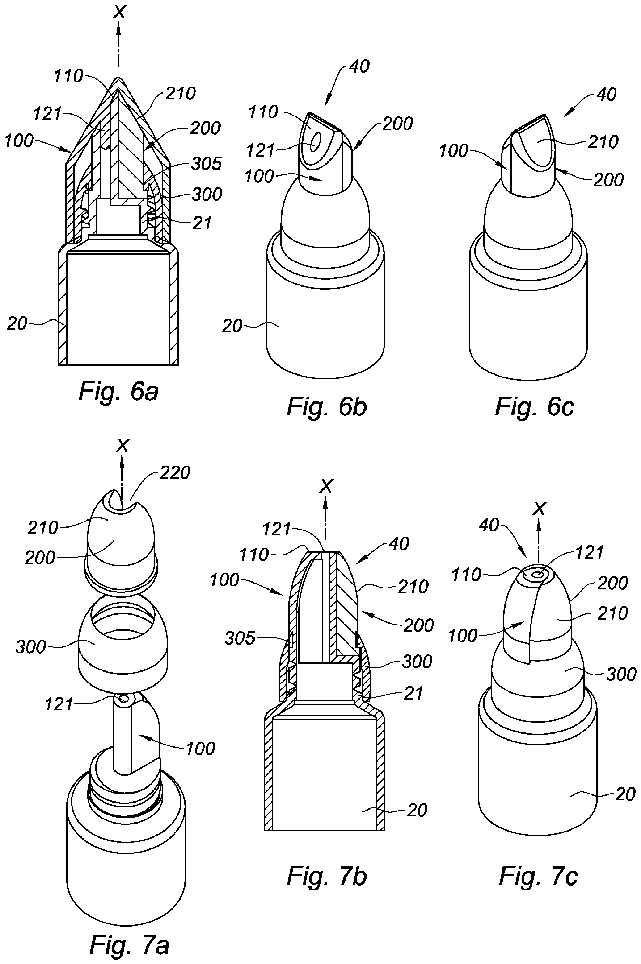

En référence aux figures 6a à 6c, la tête d'application 40 est formée par un embout de distribution 100 et un embout 200 de stockage thermique disposés de part et d'autre d'un plan longitudinal médian symétrique.

Les deux embouts de distribution 100 et de stockage thermique 200 présentent, respectivement, une face 110 de distribution et d'application 210 sous forme de biseaux orientés dans deux directions différentes et séparées par une arête d'extrémité supérieure.

Chaque embout 100,200 présente, en section transversale, une section en demi-cercle complémentaire.

Le double biseau permet d'éviter tout contact froid lors de la distribution et l'application initiale du produit sur la peau d'un utilisateur.

Pour se procurer une sensation de froid, il convient de tourner l'applicateur de 180° autour de l'axe X.

En référence aux figures 7a à 7c, la tête d'application 40 présente deux embouts 100,200 complémentaires conformés pour assurer à la tête 40 une forme générale d'ogive tronquée, à son extrémité supérieure, selon un plan perpendiculaire à l'axe X.

Cette ogive 40 présente une section transversale circulaire.

L'embout de distribution 100 présente, quant à lui, une section transversale triangulaire, au sommet arrondi.

La face de distribution 110 est confondue avec le plan tronqué de l'applicateur 20.

L'orifice de distribution 121 débouche sur cette face de distribution 110 au sommet de l'applicateur 20.

Concernant l'embout de stockage thermique 200, il présente une face d'application 210 active définie par la circonférence externe du quartier d'ogive qu'il forme.

Dès lors, les faces d'application 210 et de distribution 110 ne sont pas incluses l'une dans l'autre et sont disjointes par la présence d'une rupture de pente et un décalage axial et angulaire selon l'axe X .

Ainsi, pour obtenir la sensation de froid après une distribution et une application initiale de produit sur une zone traitée, il est nécessaire d'effectuer une rotation de l'applicateur 10 selon un axe perpendiculaire à l'axe X.

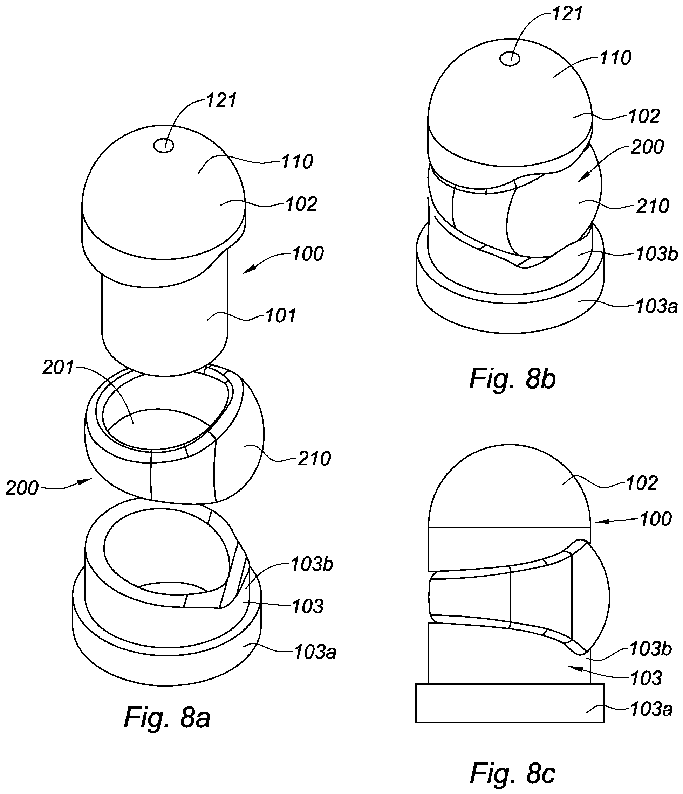

Une variante complémentaire est illustrée sur les figures 8a à 8c.

Dans cette variante, l'embout de distribution 100 prend la forme de deux éléments distincts, à savoir un premier élément tubulaire 101 prolongé à son extrémité supérieure d'une calotte 102 sphérique et un premier cylindre 103a prolongé par un second cylindre 103b de diamètre réduit, le double cylindre 103 étant ouvert à chaque extrémité.

Le canal de distribution 122 est ménagé au sein de l'élément tubulaire 101 et débouche sur un orifice 121 au sommet de la calotte sphérique 101.

Dans ce cadre, la face de distribution 110 active est définie par la surface externe de la calotte sphérique 101.

Concernant l'embout de stockage thermique 200, il comprend un bague munie sur une partie de sa circonférence d'une surface bombée définissant la face d'application thermique 210.

Cette bague est intercalée entre les deux éléments 101,103 constitutifs de l'embout de distribution 100.

L'élement tubulaire 101 de l'embout de distribution 100 est monté, de façon amovible, dans un orifice traversant central 201 de cette bague.

La bague est, dès lors, montée à la périphérie externe de l'élément tubulaire 101, sous la calotte sphérique 102.

Ainsi, les deux faces de distribution 110 et d'application 210 sont décalées angulairement, de façon non limitative, de l'ordre de 90 degrés, ce qui permet d'appliquer initialement le produit sur une zone traitée sans ressentir aucune sensation de froid.

La conformation de l'embout de stockage thermique 200 permet, avantageusement, à l'utilisateur, de porter l'embout 200 comme une bague soit au titre d'un bijou soit pour masser plus facilement une zone particulière de sa peau.

Par ailleurs, l'embout de distribution 200 est réalisé dans une matière inerte d'un point de vue chimique vis-à-vis de la peau ou du produit contenu dans le récipient 20 et d'un point de vue thermique en procurant une sensation neutre au contact de la zone traitée de l'utilisateur.

Une telle matière peut être, de façon non limitative, une matière plastique.

Dans des exemples non limitatifs de la présente invention, il peut être formé dans une matière choisie parmi le groupe suivant : polyéthylène haute ou basse densité (PEHD ou PEBD), polypropylène (PP), Polyéthylène téréphtalate (PET), polystyrène (PS), acrylonytrile butadiène styrène (ABS), styrène acrylonitryle (SAN), Polyacétal (POM), SEBS Poly(Styrene-Ethylene-Butadiene-Styrene), éthylène-propylène-diène monomère EPDM, caoutchouc, silicone, élastomères thermoplastiques, ou toute autre matière plastique souple.

Concernant l'embout de stockage thermique 200, il est réalisé dans une matière qui permet de garantir un effet de froid à l'application lié à l'effusivité thermique de la matière.

Dans des exemples non limitatifs de la présente invention, il peut être formé dans une matière choisie parmi le groupe suivant : matériaux métalliques (acier, cuivre, aluminium, titane), matériaux céramiques, oxydes métalliques (oxyde d'aluminium ou alumine, oxyde de zyrconium ou zyrcone), ou autres céramiques techniques, verre, matériaux composites, matériau minéral comme, par exemple, le marbre.

Ce peut être, en outre, une pièce de matériau quelconque recouvert d'un matériau à forte effusivité thermique comme un reventemeent métallique par procédé de galvanisation métallisation anodisation ou bien une enduction d'émail.

Par ailleurs, l'embout de stockage thermique 200 peut être massif ou recouvert en surface d'un film ou d'un revêtement d'une telle matière, voire formé partiellement d'une telle matière.

Par ailleurs, ceci afin de prolonger au maximum le contact froid sur la zone traitée, les embouts de distribution 100 et de stockage thermique 200 et, plus particulièrement, les surfaces enveloppes des faces de distribution 110 et d'application 210 occupent des volumes, respectivement, minimal et maximal.

Par ailleurs, en référence aux figures 1 et 4 à 7, le réservoir 20 peut être de tout type connu.

Il peut, ainsi, être formé d'un tube rigide ou souple.

L'applicateur 10 peut comprendre, en outre, un mécanisme de distribution de produit (non illustré) pour distribuer le produit par l'applicateur 10, le mécanisme de distribution de produit comprenant un mécanisme de distribution par mouvement de cliquet, par compression du tube, par aérosol ou piston.

De plus, le récipient 20 peut comporter ou non un goulot 21 réduit relativement aux dimensions du récipient 20, sous forme de jupe cylindrique, ouvert.

Ce goulot 21 coopère avec la tête d'application 40 pour assurer le montage de la tête sur le réservoir 20 et la communication fluidique entre le réservoir 20 et le canal de distribution 122 de l'embout de distribution 100.

Ce goulot 21 peut être formé d'une seule pièce avec le réservoir 20 ou rapporté sur ce dernier.

Concernant le montage de la tête d'application 40 sur le corps du réservoir 20, plusieurs variantes de réalisation appropriées sont illustrées sur les figures 1 à 4 et 6 à 7.

Ainsi, on peut prévoir de monter chaque embout de distribution 100 et embout de stockage thermique 200 sur le corps du récipient 20 par encliquetage, collage, sertissage, serrage, vissage, nervures ou ardillons de retenue sur l'intérieur de chaque embout de stockage thermique 200 et/ou de distribution 100 et /ou sur l'extérieur du goulot 21 et/ou par moulage en une seule pièce et/ou par l'intermédiaire d'une bague de fixation 300 ou par n'importe quels autres moyens de fixation appropriés.

En référence à la variante de réalisation des figures 1, l'embout de distribution 100 est monté, fixe, par serrage élastique du canal de distribution 122, en forme de puits, dans le goulot 21.

De plus, le système de fixation propose une bague de fixation 300 présentant une filetage 310 complémentaire d'un filetage externe ménagé d'une part, sur une jupe 230 de l'embout de stockage thermique 200 destinée à être montée à la périphérie externe du goulot 21 et, d'autre part, d'un filetage externe 22 du goulot 21.

En référence à la variante de réalisation des figures 2, l'embout de distribution 100 est moulé en une seule pièce avec le réservoir 20.

Ceci permet en effet de s'affranchir des problèmes d'étanchéité entre les deux pièces et également de réaliser des économies de fabrication et d'outillage.

L'embout de stockage thermique 200 est, quant à lui, retenu sur le goulot 21 par une bague de fixation 300 adaptée, configurée pour être montée à mouvement hélicoidal sur la périphérie externe du goulot 21, par des filetages complémentaires ménagés sur le goulot 21 et la bague 300.

La variante de réalisation des figures 3 diffère de la variante de réalisation des figures 2 en ce que l'embout de stockage thermique 200 est conformé pour être inséré dans un évidement 23 fermé, de forme complémentaire, du goulot 21.

Il est, de plus, retenu par une bague de fixation 300 montée ajustée sur la périphérie du goulot 21 de manière à interdire tout mouvement en translation de l'embout de stockage thermique 200.

Sur les figures 4, les embouts de stockage thermique 200 et de distribution 100 sont collés sur le réservoir 20.

Sur les deux variantes de réalisation illustrées sur les figures 6 et 7, chaque embout 100,200 est, retenu par une bague de fixation 300 montée sur la périphérie externe du goulot 21 par des filetages complémentaires de manière à interdire tout mouvement en translation des embouts 100,200.

Elle comprend une jupe 305 cylindrique interne adaptée pour former butée à un épaulement externe ménagé à la périphérie de chaque embout 100,200, à leur extrémité en regard du réservoir 20.

En variante, la fixation de la bague 300 peut être faite par encliquetage ou par la coopération d'une ou plusieurs rainures adaptées pour coopérer avec des ergots complémentaires.

L'applicateur 10 doit, par ailleurs, présenter une étanchétié optimale.

Dans ce cadre, diverses moyens d'étanchéité peuvent être proposés en combinaison ou non.

On peut citer, notamment, des configurations particulières du canal de distribution 122 et de l'orifice du goulot 21 du réservoir 20 adaptées pour coopérer, de manière étanche de sorte que le produit ne puisse sortir sous l'effet de la pression.

On peut, également, prévoir la mise en place de joints d'étanchéité (non illustrés sur les figures) entre le réservoir 20 et l'embout de distribution 100.

On peut citer la mise en place d'un joint dont la forme vient épouser ou s'écraser sur une forme coopérante du réservoir 20 ou un joint enserrant l'embout de distribution 100 de manière adaptée pour faire étanchéité avec l'orifice de sortie du réservoir 20.

En complément, on pourra, également, prévoir des moyens de fermeture du ou des canaux 122 d'alimentation en produit en cas de non utilisation de l'applicateur 20.

On pourra, notamment, mettre en oeuvre une bague de commande montée mobile sur le réservoir entre une position fermée où elle obture l'orifice de sortie du réservoir 20 et une position ouverte où elle libère l'orifice de sortie de ce dernier par rotation, par traction ou tout autre système d'ouverture/fermeture.

Comme indiqué précédemment, on peut, en outre, prévoir un capot de fermeture 30 configuré pour coiffer l'applicateur 20.

Ce capot 30 peut avoir toute forme souhaitée.

Il est adapté pour être monté, de manière amovible, par vissage, par encliquetage, par serrage ou tout autre moyen de fixation approprié sur le réservoir 20 et/ou la tête d'application 40, de manière à être aisément retiré et replacé par l'utilisateur tout en assurant une fermeture étanche de l'applicateur, le cas échéant.

De tels capots 30 de fermeture sont illustrés à titre d'exemples sur les figures 2a et 3b.

En référence à ces figures, on observe que le capot 30 de fermeture peut comprendre un picot 31 d'obturation adapté pour obturer l'orifice 121 débouchant sur la face de distribution 110 de l'embout de distribution 100.

Ce picot 31, de forme cylindrique, vient pénétrer dans l'orifice 121, ceci afin de maintenir une étanchéité.

Les pertes de produit en dehors de l'utilisation de l'applicateur 20 sont, dès lors, évitées.

Bien entendu, l'applicateur 10 peut contenir tout type de produit fluide liquide, semi-liquide ou visqueux.

On peut citer, notamment mais non exclusivement, des produits cosmétiques, soins contour des yeux, anticernes, produits médicaux, maquillage (rouge à lèvre notamment), soins capillaires ou colorations pour cheveux.

Bien sûr, l'invention n'est pas limitée aux exemples qui viennent d'être décrits et de nombreux aménagements peuvent être apportés à ces exemples sans sortir du cadre de l'invention.

Il est, ainsi, à noter que dans l'ensemble des variantes de réalisations décrites, il peut être envisagé de remplacer ou compléter les faces d'application 210 et de distribution 110 de surface lisse, par des surfaces plus rugueuses ou par un rêvetement de type mousse, feutre, brosse.

Par ailleurs, il peut être envisagé de ménager sur la tête d'application 40 des éléments d'application mobiles de type rouleau, éléments vibrants, électrodes ou tout autre élement adapté pour stimuler les zones traitées et/ou d'améliorer la pénétration ou l'efficacité du produit appliqué.

## Revendications

**1.** Applicateur (10) de produit fluide tel qu'un produit cosmétique, du type comprenant un réservoir (20) contenant le produit et une tête d'application (40), couplée au réservoir (20), comprenant :

- un embout de distribution (100) du produit en communication avec le réservoir (20) comportant une face (110) de distribution du produit sur une surface à traiter telle que la peau d'un utilisateur, cet embout (100) définissant une voie de distribution dudit produit en communication avec le réservoir (20), la voie de distribution du produit s'étendant à travers l'embout (100) de distribution et se terminant par un orifice (121) sur la face (110) de distribution, adapté pour distribuer le produit sur la surface à traiter,
- un embout (200) de stockage thermique comportant une face (210) d'application sur la surface à traiter,
- l'embout (200) de stockage thermique est formé d'un matériau dont l'effusivité thermique est supérieure à celle de l'embout (100) de distribution de produit de manière à procurer deux sensations thermiques différentes en contact avec la surface à traiter et,

l'applicateur (10) étant **caractérisé en ce que** :

- la tête (40) d'application est conformée de sorte que, en orientant l'applicateur (10) de façon sélective relativement à la surface à traiter, un utilisateur peut placer cette dernière en contact avec la face (110) de distribution de l'embout (100) de distribution tout en excluant un contact avec la face (210) d'application de l'embout (200) de stockage thermique, les faces de distribution (110) et d'application (210) respectives de l'embout (100) de distribution et l'embout (200) de stockage thermique étant définies par des surfaces enveloppes respectives non incluses l'une dans l'autre et disjointes.

**2.** Applicateur selon la revendication 1 dans lequel la face d'application (210) active est décalée relativement à la face de distribution (110) le long d'un axe longitudinal de l'applicateur (10) et/ou angulairement autour de la périphérie dudit applicateur (10).

**3.** Applicateur selon la revendication 1 dans lequel une rupture de pente et/ou un décrochement sont ménagés entre les faces de distribution et d'application.

**4.** Applicateur selon la revendication 1 dans lequel l'embout (200) de stockage thermique et l'embout (100) de distribution forment deux quartiers complémentaires du contour externe de l'applicateur (10) sur au moins une partie de la longueur de ce dernier.

**5.** Applicateur selon la revendication 4 dans lequel l'embout (200) de stockage thermique s'étend sur une première partie du contour périphérique externe de l'applicateur (10) et l'embout (100) de distribution s'étend sur une deuxième partie du contour périphérique de l'applicateur (10), de façon complémentaire à l'embout (200) de stockage thermique.

**6.** Applicateur selon la revendication 5 dans lequel l'embout (100) de distribution présente une forme de canule (130) longiligne terminée par l'orifice de distribution (121).

**7.** Applicateur selon l'une des revendications 5 à 6 dans lequel l'orifice de distribution (121) est déporté à la périphérie externe de la face d'application de l'embout de stockage thermique et/ou en saillie de la tête d'application.

**8.** Applicateur selon la revendication 1 dans lequel l'embout de stockage thermique (200) est formé par une bague munie d'une face d'application bombée, adaptée pour être montée, de façon amovible, sur l'embout de distribution.

## Patentansprüche

**1.** Applikator (10) für ein fluides Produkt, wie etwa ein Kosmetikprodukt, von dem Typ, der einen das Produkt enthaltenden Behälter (20) und einen mit dem Behälter (20) gekoppelten Applikationskopf (40) umfasst, umfassend:

- ein mit dem Behälter (20) in Kommunikation stehendes Endstück zum Ausgeben (100) des Produkts, das eine Seite (110) zum Ausgeben des Produkts auf eine zu behandelnde Fläche, wie etwa die Haut eines Benutzers, umfasst, wobei dieses Endstück (100) einen mit dem Behälter (20) in Kommunikation stehenden Weg zum Ausgeben des Produkts definiert, wobei sich der Weg zum Ausgeben des Produkts durch das Ausgabe-Endstück (100) hindurch erstreckt und auf der Ausgabeseite (110) mit einer Öffnung (121) endet, die dafür geeignet ist, das Produkt auf die zu behandelnde Fläche auszugeben,
- ein Wärmespeicher-Endstück (200), das eine Seite (210) zum Applizieren auf die zu behandelnde Fläche umfasst,
- das Wärmespeicher-Endstück (200) aus einem Material gebildet ist, dessen Wärmeeindringkoeffizient größer ist als derjenige des Produktausgabe-Endstücks (100), um bei Berührung mit der zu behandelnden Fläche zwei unterschiedliche Wärmeempfindungen zu bieten, und

wobei der Applikator (10) **dadurch gekennzeichnet ist, dass**:

- der Applikationskopf (40) so ausgebildet ist, dass ein Benutzer durch selektives Ausrichten des Applikators (10) relativ zu der zu behandelnden Fläche diese letztere mit der Ausgabeseite (110) des Ausgabe-Endstücks (100) in Berührung bringen kann, während gleichzeitig eine Berührung mit der Applikationsseite (210) des Wärmespeicher-Endstücks (200) ausgeschlossen wird, wobei die jeweilige Ausgabe- (110) und Applikationsseite (210) des Ausgabe-Endstücks (100) und des Wärmespeicher-Endstücks (200) von jeweiligen Hüllflächen definiert werden, die nicht ineinander eingeschlossen und getrennt sind.

2. Applikator nach Anspruch 1, wobei die aktive Applikationsseite (210) relativ zur Ausgabeseite (110) entlang einer Längsachse des Applikators (10) und/oder winkelmäßig um den Umfang des Applikators (10) herum versetzt ist.

3. Applikator nach Anspruch 1, wobei zwischen der Ausgabe- und Applikationsseite ein Gefällsbruch und/oder ein Absatz ausgestaltet sind.

4. Applikator nach Anspruch 1, wobei das Wärmespeicher-Endstück (200) und das Ausgabe-Endstück (100) zwei komplementäre Bezirke der Außenkontur des Applikators (10) auf mindestens einem Teil der Länge dieses letzteren bilden.

5. Applikator nach Anspruch 4, wobei sich das Wärmespeicher-Endstück (200) auf einem ersten Teil der Außenumfangskontur des Applikators (10) erstreckt, und sich das Ausgabe-Endstück (100) komplementär zum Wärmespeicher-Endstück (200) auf einem zweiten Teil der Umfangskontur des Applikators (10) erstreckt.

6. Applikator nach Anspruch 5, wobei das Ausgabe-Endstück (100) eine längliche Kanülenform (130) aufweist, die mit der Ausgabeöffnung (121) endet.

7. Applikator nach einem der Ansprüche 5 bis 6, wobei die Ausgabeöffnung (121) an den Außenumfang der Applikationsseite des Wärmespeicher-Endstücks verschoben ist und/oder vom Applikationskopf vorspringt.

8. Applikator nach Anspruch 1, wobei das Wärmespeicher-Endstück (200) von einem Ring gebildet wird, der mit einer bombierten Applikationsseite ausgestattet ist, die dafür geeignet ist, abnehmbar am Ausgabe-Endstück montiert zu werden.

**Claims**

1. An applicator (10) for a fluid product such as a cosmetic product, of the type comprising a reservoir (20) containing the product and an application head (40), coupled to the reservoir (20), comprising:

- a tip (100) for dispensing the product in communication with the reservoir (20) including a face (110) for dispensing the product on a surface to be treated such as the skin of a user, this tip (100) defining a path for dispensing said product in communication with the reservoir (20), the path for dispensing the product extending through the dispensing tip (100) and terminating in an orifice (121) on the dispensing face (110), adapted to dispense the product on the surface to be treated,
- a tip (200) for thermal storage including a face (210) for application on the surface to be treated.

the tip (200) for thermal storage is formed of a material whose thermal effusivity is higher than that of the tip (100) for dispensing the product so as to provide two different thermal sensations in contact with the surface to be treated and,
the applicator (10) being **characterized in that**:

- the application head (40) is shaped so that, by selectively orienting the applicator (10) relative to the surface to be treated, a user can place the latter in contact with the dispensing face (110) of the dispensing tip (100) while excluding any contact with the application face (210) of the thermal storage tip (200), the respective dispensing (110) and application (210) faces of the dispensing tip (100) and the thermal storage tip (200) being defined by respective envelope surfaces not included into each other and disjointed.

2. The applicator according to claim 1 wherein the active application face (210) is shifted with respect to the dispensing face (110) along a longitudinal axis of the applicator (10) and/or angularly around the periphery of said applicator (10).

3. The applicator according to claim 1 wherein a slope discontinuity and/or a step are formed between the dispensing and application faces.

4. The applicator according to claim 1 wherein the thermal storage tip (200) and the dispensing tip (100) form two complementary quarters of the outer contour of the applicator (10) over at least part of the length of the latter.

5. The applicator according to claim 4 wherein the thermal storage tip (200) extends over a first portion of the outer peripheral contour of the applicator (10) and the dispensing tip (100) extends over a second portion of the peripheral contour of the applicator (10), complementary to the thermal storage tip (200).

6. The applicator according to claim 5 wherein the dispensing tip (100) has a slender cannula shape (130) terminated by the dispensing orifice (121).

7. The applicator according to any of claims 5 to 6 wherein the dispensing orifice (121) is offset to the outer periphery of the application face of the thermal storage tip and/or projecting from the application head.

8. The applicator according to claim 1 wherein the thermal storage tip (200) is formed by a ring provided with a cambered application face, adapted to be removably mounted on the dispensing tip.

Fig. 1a

Fig. 1b

Fig. 1c

Fig. 1d

Fig. 2c

Fig. 2a

Fig. 2b

Fig. 3a

Fig. 3b

Fig. 3c

Fig. 3d

**Fig. 4a**

**Fig. 4b**

**Fig. 4c**

**Fig. 5a**

**Fig. 5b**

Fig. 6a

Fig. 6b

Fig. 6c

Fig. 7a

Fig. 7b

Fig. 7c

Fig. 8a

Fig. 8b

Fig. 8c

**EP 2 866 606 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2915972